# EUROPEAN PATENT APPLICATION

(11) **EP 4 494 631 A1**
(43) Date of publication of application: **22.01.2025**
(21) Application number: 24184573.4
(22) Date of filing: 26.06.2024
(51) Int. Cl.: A61K 8/9789, A61K 36/00, A61P 17/04, A61Q 19/08

(54) **A SENOTHERAPY COMPOSITION CONTAINING CAMELLIA SINENSIS EXTRACT**

(30) Priority: 30.06.2023 KR 20230084876
(71) Applicant: Amorepacific Corporation, Seoul 04386 (KR)
(72) Inventor: PARK, Sunyoug, Gyeonggi-do 17074 (KR); CHO, Si Young, Gyeonggi-do 17074 (KR); HWANG, Kyeonghwan, Gyeonggi-do 17074 (KR); MIN, Daejin, Gyeonggi-do 17074 (KR)
(74) Representative: Germain Maureau

(57) **Abstract**

Disclosed herein is a method for senotherapy; prevention, improvement or treatment of aging-related disease; improvement of skin elasticity or wrinkles; improvement of skin barrier; or improvement of skin pigmentation, containing administering an effective amount of tea tree (*Camellia Sinensis*) root extract to a subject in need thereof. In one aspect, a senotherapy composition containing the tea tree (*Camellia Sinensis*) root extract has an excellent senolytic effect to selectively remove senescent cells and an excellent senomorphic effect to inhibit the production of senescence associated secretory phenotype (SASP) of senescent cells.

## Description

### Field of the Invention

Disclosed herein is a senotherapy composition.

### Description of the Related Art

Compared to young cells, senescent cells have the characteristics of being less reactive and more resistant to death, making them less likely to die. Along with this resistance to death, senescent cells promote aging or deteriorate the function of surrounding cells by secreting Senescence Associated Secretory Phenotype (SASP), a complex of various aging promoting factors. For this reason, preventing the spread of SASP is a major challenge in anti-aging. In addition, substances that induce selective death of senescent cells are considered good drug candidates for preventing age-related diseases or preventing a deterioration of cell function. The senescent cell-selective death-inducing substances developed to date are highly cytotoxic, so most of them are medicines containing anticancer drugs that cannot be used as cosmetic raw materials. The problem is that only a few of them are effective against skin cells.

Senotherapy is a treatment-related therapy that targets senescent cells, including a senolytic that selectively removes senescent cells and a senomorphic that prevents the production of SASP in senescent cells. The senolytic induces apoptosis of senescent cells, and ABT737, a Bcl-2 inhibitor, is a representative example.

### SUMMARY OF THE INVENTION

One object of the present disclosure is to provide a senolytic composition.

Another object of the present disclosure is to provide a senomorphic composition.

In order to achieve the above object, in one aspect, the present disclosure provides a senolytic composition comprising a tea tree root extract as an active ingredient.

In another aspect, the present disclosure provides a senomorphic composition comprising a tea tree root extract as an active ingredient.

### Advantageous Effects of Invention

In one aspect, the composition of the present disclosure has excellent senolytic efficacy for selectively removing senescent cells.

In another aspect, the composition of the present disclosure has excellent senomorphic efficacy that inhibits the production of senescence associated secretory phenotype (SASP) in senescent cells.

In another aspect, the composition of the present disclosure can improve skin elasticity or wrinkles due to the senolytic and senomorphic effects, and can further be used to prevent, improve, and treat aging-related diseases.

In another aspect, the composition of the present disclosure has excellent skin barrier improvement efficacy.

In another aspect, the composition of the present disclosure has excellent skin pigmentation improvement efficacy.

### BRIEF DESCRIPTION OF THE DRAWINGS

FIG. 1 relates to the senolytic efficacy of tea tree (*Camellia Sinensis*) root extract.
FIGs. 2A and 2B show the increase in apoptosis of senescent cells by treatment with tea tree root extract.
FIG. 3 relates to the senomorphic efficacy of the tea tree root extract. Specifically, it shows the decrease in the expression of genes related to senescence associated secretory phenotype (SASP) due to treatment with tea tree root extract.
FIGs. 4A and 4B show the reduction of beta-galactosidase (β-galactosidase), an aging factor, by treatment with tea tree root extract.
FIGs. 5A to 5C show the recovery of elasticity (A), dermis (B) and epidermal function (C) in an artificial skin model by treatment with tea tree root extract.
FIGs. 6A and 6B show the negative effects of SASP secreted from senescent cells on surrounding keratinocytes and the inhibition of SASP at the protein level (A) and mRNA (B) levels by treatment with tea tree root extract.
FIGs. 7A and 7B show the results of analyzing the catechin (A) and amino acid (B) content and components of the tea tree root extract.
FIG. 8 relates to the toxicity of young fibroblasts by treatment with epigallocatechin gallate (EGCG).
FIGs. 9A and 9B relate to the efficacy of improving skin pigmentation by treatment with tea tree root extract.

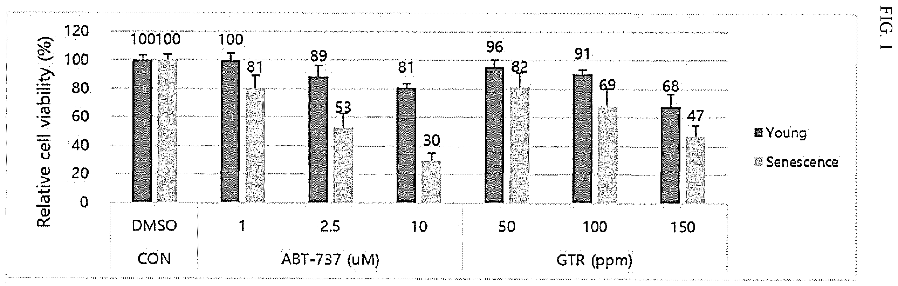

In FIGs. 1 to 9, GTR represents a tea tree root extract.

### DETAILED DESCRIPTION OF THE INVENTION

Hereinafter, the present disclosure will be described in detail.

As used herein, the term "cellular senescence" refers to a state in which normal cells lose the ability to divide and cell cycle arrest continues. The cellular senescence may progress into a stage in which cell division stops, a stage in which senescence spreads when cells that have stopped dividing secrete a senescence associated secretory phenotype (SASP), and a stage in which senescent cells accumulate in tissues.

In this specification, the term "senolytic" is a compound word combining senescence (aging) and lytic (destroy), and refers to a material that selectively removes senescent cells.

In this specification, the term "senomorphic" is a compound word combining senescence (aging) and morphic (shape), and refers to a material that returns to normal cell levels by inhibiting the secretion of specific factors, including inhibition of senescence associated secretory phenotype (SASP) factors.

In one aspect, the present disclosure relates to a senolytic composition comprising a tea tree (*Camellia Sinensis*) root extract as an active ingredient.

The tea tree (*Camellia Sinensis*) root extract usually contains oleanane saponin as a main ingredient (e.g., 53% of the total content). The components of tea tree root extract are completely different from those of tea tree leaf extract. The tea tree leaf extract has a catechin content of about 30% by weight, while the tea tree root extract has a catechin content of only about 0.2% by weight. This means that the efficacy of catechin cannot be expected from the tea tree root extract.

In one exemplary embodiment, the senolytic may be a skin cell senolytic.

In one exemplary embodiment, the skin cells may be fibroblasts.

In an exemplary embodiment, the senolytic may be a senolytic based on apoptosis.

In an exemplary embodiment, the tea tree root extract may be obtained according to a method known in the art. Specifically, the tea tree roots are washed with purified water, dried by sun drying or hot air drying, and powdered. An extraction solvent can be used in the granulated power, and the extraction solvent may be selected from organic solvents such as ethanol, methanol, butanol, ether, ethyl acetate, chloroform, or a mixed solvent of these organic solvents and water. Considering the safety of the raw materials, water or ethanol at a concentration of 30 to 70% is preferred. A dried extract of the tea tree root may be obtained by refluxing extraction the extract obtained by using the solvent above, filtering, and concentrating under reduced pressure at 40 to 50°C.

In one exemplary embodiment, the composition may be administered to a subject in need of restoring skin elasticity or improving wrinkles.

In one exemplary embodiment, the composition may be administered to a subject in need of a decrease in MMP-1 or an increase in procollagen in the dermal layer of the skin.

In one exemplary embodiment, the composition may be administered to a subject in need of cell production, proliferation, or differentiation of the epidermal layer of the skin.

In one exemplary embodiment, the composition may be administered to a subject in need of production, delivery, or decomposition of skin pigment in the epidermal layer of the skin.

In an exemplary embodiment, the composition may be administered to a subject in need of inhibiting the production and transfer of melanin and decomposing it in the epidermal layer of the skin.

In another aspect, the present disclosure relates to a method for selectively removing senescent cells by administering an effective amount of a tea tree root extract to a subject.

In another aspect, the present disclosure relates to the use of the tea tree root extract for manufacturing a senolytic composition.

In another aspect, the present disclosure relates to the use of the tea tree root extract for a senolytic.

In another aspect, the present disclosure relates to a senomorphic composition comprising a tea tree root extract as an active ingredient.

In one exemplary embodiment, the senomoltic may be a skin cell senomoltic.

In one exemplary embodiment, the skin cells may be fibroblasts.

In an exemplary embodiment, the tea tree root extract may be obtained according to a method known in the art. Specifically, the tea tree roots are washed with purified water, dried by sun drying or hot air drying, and powdered. An extraction solvent can be used in the granulated power, and the extraction solvent may be selected from organic solvents such as ethanol, methanol, butanol, ether, ethyl acetate, chloroform, or a mixed solvent of these organic solvents and water. Considering the safety of the raw materials, water or ethanol at a concentration of 30 to 70% is preferred. A dried extract of the tea tree root may be obtained by refluxing extraction the extract obtained by using the solvent above, filtering, and concentrating under reduced pressure at 40 to 50°C.

In one exemplary embodiment, the composition may be administered to a subject in need of restoring skin elasticity or improving wrinkles.

In one exemplary embodiment, the composition may be administered to a subject in need of a decrease in MMP-1 or an increase in procollagen in the dermal layer of the skin.

In one exemplary embodiment, the composition may be administered to a subject in need of cell production, proliferation, or differentiation of the epidermal layer of the skin.

In one exemplary embodiment, the composition may be administered to a subject in need of production, delivery, or decomposition of skin pigment in the epidermal layer of the skin.

In an exemplary embodiment, the composition may be administered to a subject in need of inhibiting the production and transfer of melanin and decomposing it in the epidermal layer of the skin.

In an exemplary embodiment, the composition may be administered to a subject in need of reducing aging factors or senescence associated secretory phenotype (SASP) in the skin. The aging factor may be senescence associated beta-galactosidase (SA-β), and the SASP may be one or more selected from the group consisting of IL-6, IL-8, MMP-1, and MCP-1.

In another aspect, the present disclosure relates to a method for reducing aging factors or senescence associated secretory phenotype (SASP) by administering an effective amount of a tea tree root extract to a subject.

In another aspect, the present disclosure relates to the use of the tea tree root extract for manufacturing a senomorphic composition.

In another aspect, the present disclosure relates to the use of the tea tree root extract for a senomorphic.

In an exemplary embodiment, the daily administration amount of the active ingredient of the composition may be 10µg/kg to 100 g/kg, and more specifically, 10 mg/kg or more, 15 mg/kg or more, 25 mg/kg or more, 30 mg/kg or more, 35 mg/kg or more, 40 mg/kg or more, 45 mg/kg or more, or 50 mg/kg or more, 200 mg/kg or less, 190 mg/kg or less, 180 mg/kg or less, 170 mg/kg or less, 165 mg/kg or less, 160 mg/kg or less, 155 mg/kg or less, or 150 mg/kg or less. The efficacy of senolytic or senomorphic is excellent at the above administration amount, the efficacy of senolytic or senomorphic is minimal when the administration amount is less than the above administration amount. It the administration amount is higher than the above, problems such as toxicity may occur. The administration may be administered once or several times a day. For example, it may be administered 1 to 12 times a year 2 to 24 times per day, 1 to 2 times per 3 days, 1 to 6 times per week, 1 to 10 times per 2 weeks, 1 to 15 times per 3 weeks, 1 to 3 times per 4 weeks, or 1 to 12 times per year.

In another aspect, the present disclosure relates to a composition for preventing, improving, or treating aging-related diseases, comprising a tea tree root extract as an active ingredient.

In an exemplary embodiment, the aging-related disease may be a skin aging-related disease, and may include, for example, pruritus, dry skin, bedsores, pigmentary disease, benign tumor, skin cancer, etc.

In another aspect, the present disclosure relates to a composition for improving skin elasticity or wrinkles comprising a tea tree root extract as an active ingredient. Due to the aging of skin cells, especially the decrease in elastin and collagen produced by fibroblasts in the dermal tissue, wrinkles, sagging symptoms, and decreased skin elasticity may occur. However, the tea tree root extract may improve these wrinkles and skin elasticity.

In another aspect, the present disclosure relates to a composition for improving the skin barrier comprising a tea tree root extract as an active ingredient.

In another aspect, the present disclosure relates to a composition for improving skin pigmentation comprising a tea tree root extract as an active ingredient.

In an exemplary embodiment, the composition may be one or more selected from the group consisting of cosmetic composition, pharmaceutical composition, and food composition.

In one exemplary embodiment, the composition may contain 0.001 to 99.9% by weight of the active ingredient. For example, it may contain 0.01 to 20.0% by weight or 0.1 to 10.0% by weight.

In an exemplary embodiment, the cosmetic composition may be manufactured in the form of a general emulsified formulation and solubilized formulation. The emulsified formulations include nourishing lotions, creams, essences, etc., and the solubilized formulations include softening lotions, etc. Suitable formulations may be in form of, solutions, gels, solid or pasty anhydrous products, emulsions obtained by dispersing the oil phase in the aqueous phase, suspensions, microemulsions, microcapsules, microgranules or ionic (liposome) or bion-type vesicular dsipersants, creams, skins, lotions, powders, ointments, sprays or conceal sticks. It may also be in the form of foam or an aerosol composition further containing compressed propellant.

In an exemplary embodiment, the cosmetic composition may additionally contain fatty substances, organic solvents, solubilizers, thickening and gelling agents, softeners, antioxidants, suspending agents, stabilizers, foaming agents, fragrances, surfactants, water, ionic type. or non-ionic emulsifiers, fillers, sequestering agents, chelating agents, preservatives, vitamins, blocking agents, wetting agents, essential oils, dyes, pigments, hydrophilic or lipophilic active agents, lipid vesicles or commonly used adjuvants such as other ingredients commonly used in cosmetic compositions.

In an exemplary embodiment, the pharmaceutical composition may be provided in any formulation suitable for topical administration. For example, it can be administered orally, transdermally, intravenously, intramuscularly, or by subcutaneous injection. As an example, the pharmaceutical composition may be an injection, a solution for external use on the skin, a suspension, an emulsion, a gel, a patch, or a spray. The formulation may be easily manufactured according to conventional methods in the field, and surfactants, excipients, wetting agents, emulsification accelerators, suspending agents, salts or buffers for adjusting osmotic pressure, colorants, flavorings, stabilizers, preservatives, preservatives or other commonly used supplements may be used appropriately.

In an exemplary embodiment, the active ingredient of the pharmaceutical composition will vary depending on the subject's age, gender, weight, pathological condition and its severity, route of administration, or the judgment of the prescriber. Determination of the appropriate dosage to use based on these factors is within the level of one skilled in the art.

In an exemplary embodiment, when the composition is used as an additive to a health functional food, it may be added as is or used together with other foods or food ingredients, and may be used appropriately according to conventional methods. The mixed amount of active ingredients may be appropriately determined depending on each purpose of use, such as prevention, health, or treatment. The formulation of health functional foods may be in the form of powders, granules, pills, tablets, capsules, as well as general foods or beverages.

In an exemplary embodiment, there is no particular limitation on the type of the health functional food, and examples of foods to which the composition may be added may include meat, confectionery, noodles, gum, dairy products including ice cream, various soups, beverages, tea, drinks, alcoholic beverages, and vitamin complexes, etc., and may include all foods in the conventional sense.

In an exemplary embodiment, the beverage among the health functional foods may contain various flavoring agents or natural carbohydrates as additional ingredients like a typical beverage. The above-mentioned natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, natural sweeteners such as thaumatin and stevia extract or synthetic sweeteners such as saccharin and aspartame may be used. The ratio of the natural carbohydrate may be about 0.01 to 0.04 g, preferably about 0.02 to 0.03 g, per 100 mL of the beverage according to the present disclosure.

In an exemplary embodiment, in addition to the above, the health functional food according to the present disclosure may include various nutrients, vitamins, electrolytes, flavors, colorants, pectic acid and its salts, alginic acid and its salts, organic acids, protective colloidal thickeners, pH conditioners, stabilizers, preservatives, glycerin, alcohol, and carbonating agents used in carbonated beverages. In addition, the health functional food according to the present disclosure may include fruit juice drinks, vegetable drinks and pulp for the production of natural fruit juice. These ingredients may be used independently or in combination. The ratio of these additives is generally selected in the range of 0.01 to 0.1 parts by weight based on 100 parts by weight of the health functional food according to the present disclosure.

In another aspect, the present disclosure relates to a method for preventing, improving, or treating aging-related diseases by administering an effective amount of tea tree root extract to a subject.

In another aspect, the present disclosure relates to the use of tea tree root extract for manufacturing a composition for preventing, improving or treating age-related diseases.

In another aspect, the present disclosure relates to the use of the tea tree root extract for preventing, improving or treating age-related diseases.

In another aspect, the present disclosure relates to a method for improving skin elasticity or wrinkles by administering an effective amount of tea tree root extract to a subject.

In another aspect, the present disclosure relates to the use of tea tree root extract for manufacturing a composition for improving skin elasticity or wrinkles.

In another aspect, the present disclosure relates to the use of the tea tree root extract for improving skin elasticity or wrinkles.

In another aspect, the present disclosure relates to a method for improving the skin barrier by administering an effective amount of tea tree root extract to a subject.

In another aspect, the present disclosure relates to the use of tea tree root extract for manufacturing a composition for improving the skin barrier.

In another aspect, the present disclosure relates to the use of the tea tree root extract for improving the skin barrier.

In another aspect, the present disclosure relates to a method for improving skin pigmentation by administering to a subject comprising an effective amount of tea tree root extract.

In another aspect, the present disclosure relates to the use of tea tree root extract for manufacturing a composition for improving skin pigmentation.

In another aspect, the present disclosure relates to the use of the tea tree root extract for improving skin pigmentation.

In another aspect, the present disclosure relates to the use of the tea tree root extract for non-therapeutic use or therapeutic use.

Hereinafter, the configuration and effects of the present disclosure will be described in more detail through examples. However, the examples below are provided only for illustrative purposes to aid understanding of the present disclosure.

### [Example]

### [Example 1]

### Preparation of Tea Tree Root Extract

Tea tree (Camellia sinensis) roots purchased from Jeju Osulloc Farm's Dolsongi Tea Field, Seogwang Tea Field, and Hannam Tea Field were extracted in 70% ethanol and dried in vacuum to obtain tea tree root extract (GTR) powder.

### [Example 2]

### Results of measuring the degree of cell death depending on the treatment with tea tree root extract

Human fibroblasts were cultured in a 6-well plate culture medium. Senescent cells were prepared by inducing senescence by culturing the fibroblasts in DMEM (containing 10% FBS) medium containing doxorubicin (100 ng/ml) and insulin-like growth factor 1 (IGF-1) (100ng/ml) for 4 days. Then, the same medium was treated with a positive control material (ABT737, 1-10 uM) and the tea tree root extract (50-150ppm) and cultured for 72 hours. The ABT737 is a Bcl-2 inhibitor and a representative senolytic. Young cells were treated with experimental mmaterials in DMEM medium without inducing senescence and cultured for 72 hours.

In order to confirm the senolytic efficacy of the treatment with the tea tree root extract, the medium was replaced with a medium diluted 1/100 with Cell counting kit-8 (CCK-8, dojindo), cultured for 1 hour, and then the absorbance was measured at 540 nm.

As shown in FIG. 1, it was confirmed that the tea tree root extract almost did not kill young cells at a concentration of 50-100 ppm, but only killed about 22% and 31% of aged cells, respectively. In addition, it was confirmed that the efficacy of senescent cell death increased in a concentration-dependent manner.

### [Example 3]

### Results of measuring the degree of apoptosis in aged cells depending on the treatment with tea tree root extract

Human fibroblasts were cultured in a 6-well plate culture medium. Senescent cells were prepared by inducing senescence by culturing the fibroblasts in DMEM (containing 10% FBS) medium containing doxorubicin (100 ng/ml) and insulin-like growth factor 1 (IGF-1) (100ng/ml) for 4 days. After, the same medium was treated with a positive control material (ABT737, 10 uM) and the tea tree root extract (150ppm) and cultured for 24 hours, and then the cells were harvested. The harvested cells were treated with an apoptosis assay kit (APC annexin V/Dead cell apoptosis kit with APC annexin V and SYTOX green for flow cytometry, invitrogen) according to the user's manual, and fluorescence value of each cell was analyzed using a FACS (BD FACSuite) device.

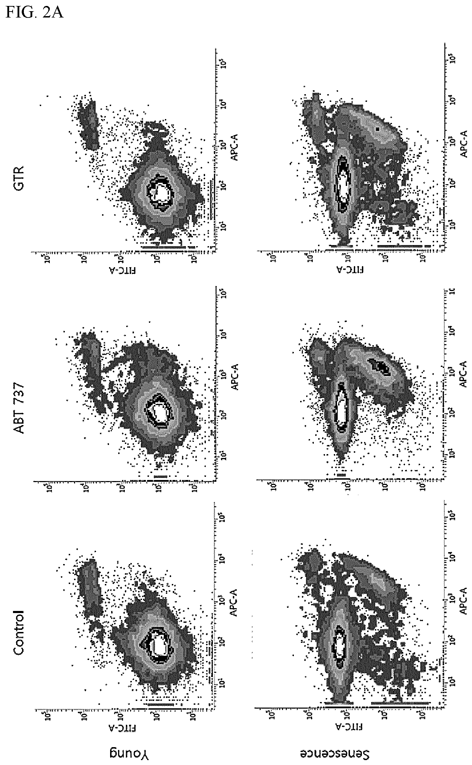

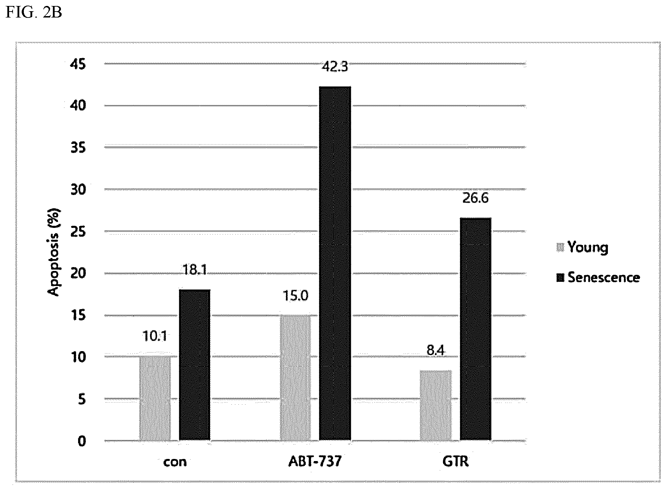

As shown in FIGs. 2A and 2B, apoptosis was slightly reduced in young cells (8.4%) compared to the negative control group (10.1%). In contrast, it was confirmed that apoptosis was significantly increased in aged cells (26.6%) compared to the negative control group (18.8%).

### [Example 4]

### Results of measuring the expression of genes related to senescence associated secretory phenotype (SASP) in aged cells depending on the treatment with tea tree root extract

Human fibroblasts were cultured in a 6-well plate culture medium. Senescent cells were prepared by inducing senescence by culturing the fibroblasts in DMEM (containing 10% FBS) medium containing doxorubicin (100 ng/ml) and insulin-like growth factor 1 (IGF-1) (100ng/ml) for 4 days. Then, the same medium was treated with a positive control material (ABT737 2.5 uM) and the tea tree root extract (50-150ppm) and cultured for 72 hours. The cells were transferred to a new 6-well plate culture medium, cultured for an additional 7 days, then harvested and genes were collected. Using 1ug of the collected mRNA, cDNA was synthesized, and real-time qPCR (thermofisher) was performed to compare the expression of genes. The genes of IL-6 (Hs00985639_m1), IL-8 (Hs00174103_m1), and MMP-1 (Hs00899658_m1) were quantified by normalization to GAPDH (Hs02786624_g1).

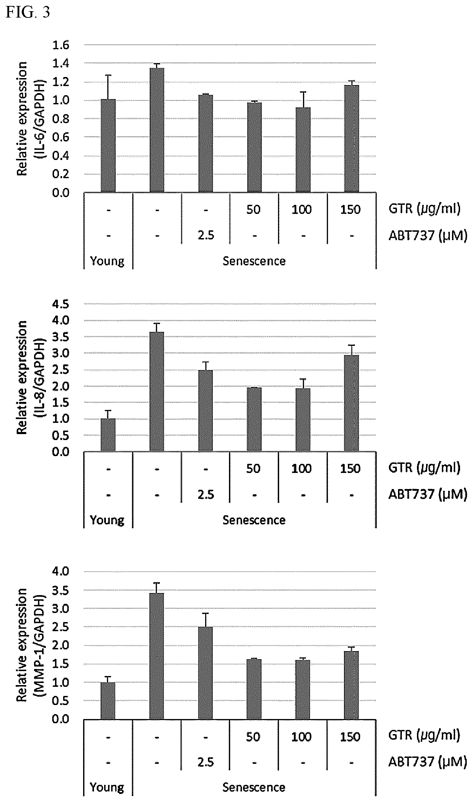

As shown in FIG. 3, it was confirmed that the expression of SASP genes (IL-6, IL-8, MMP-1) increased by senescence induction was reduced by treatment with the tea tree root extract and the level of expression was maintained even after 7 days.

### [Example 5]

### Results of measuring the expression of aging factors in aged cells depending on the treatment with tea tree root extract

Human fibroblasts were cultured in a 6-well plate culture medium. Senescent cells were prepared by inducing senescence by culturing the fibroblasts in DMEM (containing 10% FBS) medium containing doxorubicin (100 ng/ml) and insulin-like growth factor 1 (IGF-1) (100ng/ml) for 4 days. After, the same medium was treated with the tea tree root extract (10-150ppm) and cultured for 72 hours, and then the cells were harvested. The harvested cells were treated with medium diluted 1/1,000 with Spider's SPiDER-βGal (dojindo) for 15 minutes, and then the fluorescence value of each cell was measured through FACS (BD FACSuite).

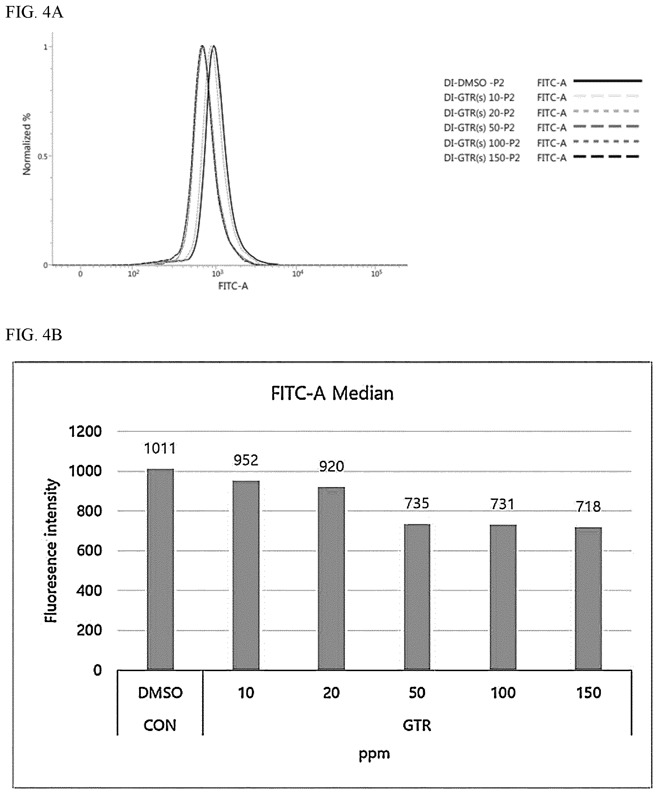

As shown in FIGs. 4A and 4B, the efficacy of beta-galactosidase (β-Galactosidase) reduction of about 30% was confirmed at a concentration of 50 ppm or more of the tea tree root extract.

### [Example 6]

### Confirmation of changes in dermal layer elasticity and epidermal layer differentiation of artificial skin model depending on the treatment with tea tree root extract

Human fibroblasts were cultured in a 100 pie culture medium. Senescent cells were prepared by inducing senescence by culturing the fibroblasts in DMEM (containing 10% FBS) medium containing doxorubicin (100 ng/ml) and insulin-like growth factor 1 (IGF-1) (100ng/ml) for 4 days. Then, the same medium was treated with the tea tree root extract (1000ppm) and cultured for 72 hours. A dermal layer was prepared by culturing 1×10⁵ fibroblasts in collagen gel. After purchasing Melanoderm (Martek), which consists of the epidermis, the dermal layer was artificially attached to the lower part thereof and cultured for two weeks. After 14 days, the culture medium was collected and the expression levels of pro-collagen and MMP-1 were analyzed using ELISA. The cultured artificial skin was confirmed to express Ki67, K10, and Flaggrin through immunostaining.

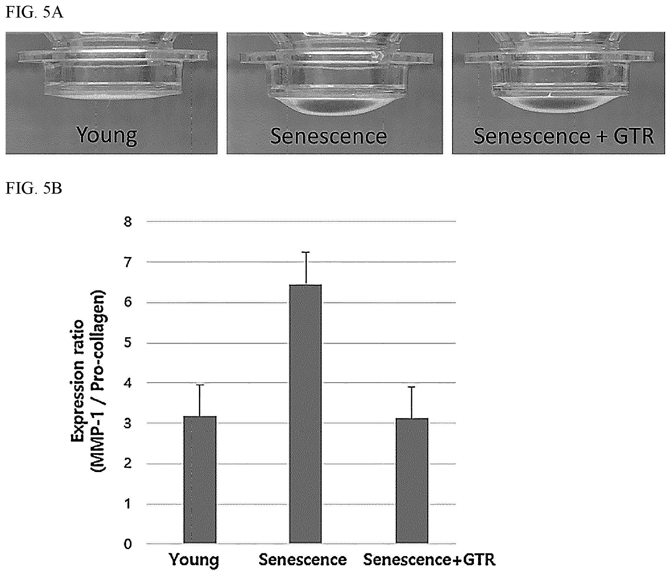

As shown in FIG. 5A, the dermal layer made of young fibroblasts had a strong pulling force on the surrounding collagen, resulting in strong contraction, but the contraction of the dermal layer made of aged fibroblasts was observed to be weak. On the other hand, it was confirmed that the dermal layer prepared from aged fibroblasts treated with the tea tree root extract had improved contractility. This was also confirmed by the recovery of the ratio of MMP-1 and pro-collagen produced and secreted in the dermal layer to normal, as shown in FIG. 5B.

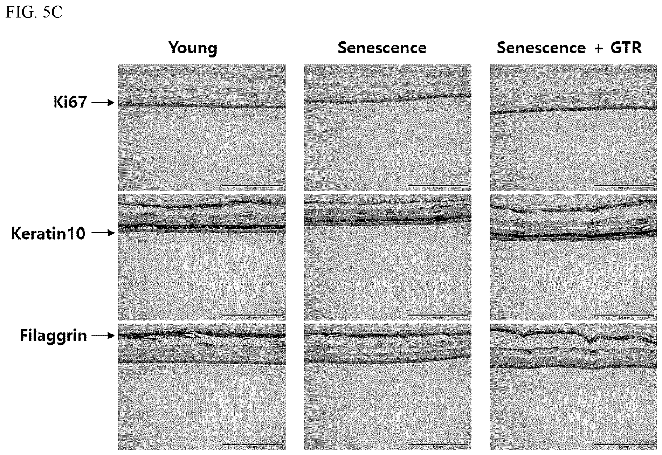

In addition, as a result of confirming the effect on the surrounding epidermal layer through immunostaining, it was confirmed that the proliferation and differentiation ability of the epidermal layer, which was reduced due to aging, was improved by treatment with the tea tree root extract, as shown in FIG. 5C.

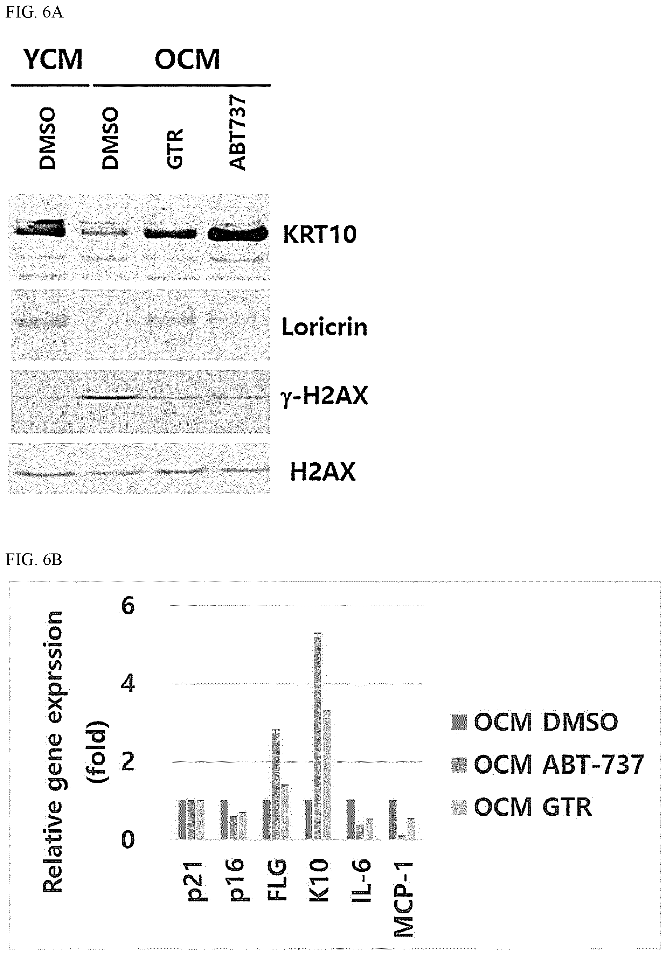

Furthermore, the negative effects of SASP on surrounding keratinocytes and the inhibitory effect of SASP at the protein (A) and mRNA (B) levels by treatment with the tea tree root extract were confirmed. When the supernatant of aged cells (old conditioned media, OCM) was treated, KRT10 and Loricrin, markers related to skin barrier, decreased, and r-H2X, a factor related to cell damage, increased compared to the control group (young conditioned media, YCM) (FIG. 6A). However, in the case of conditioned media obtained by treating aged cells with the tea tree root extract, it was confirmed that damage to the skin barrier or cells was recovered (FIG. 6B). Specifically, among aging markers, p16 decreased, FLG and K10, markers related to keratinocyte differentiation and skin barrier, increased, and IL-6 and SASPs, IL-6 and MCP-1, decreased (FIG. 6B). The senomorphic and skin barrier improving efficacy of the tea tree root extract was confirmed through a decrease in the expression of the aging and SASP markers and an increase in markers related to keratinocytes differentiation and skin barrier.

### [Example 7]

### Results of analyzing the content and component of catechin and amino acids of the tea tree root extract

A tea tree leaf extract and a tea tree root extract were analyzed for catechin and amino acid content through HPLC analysis.

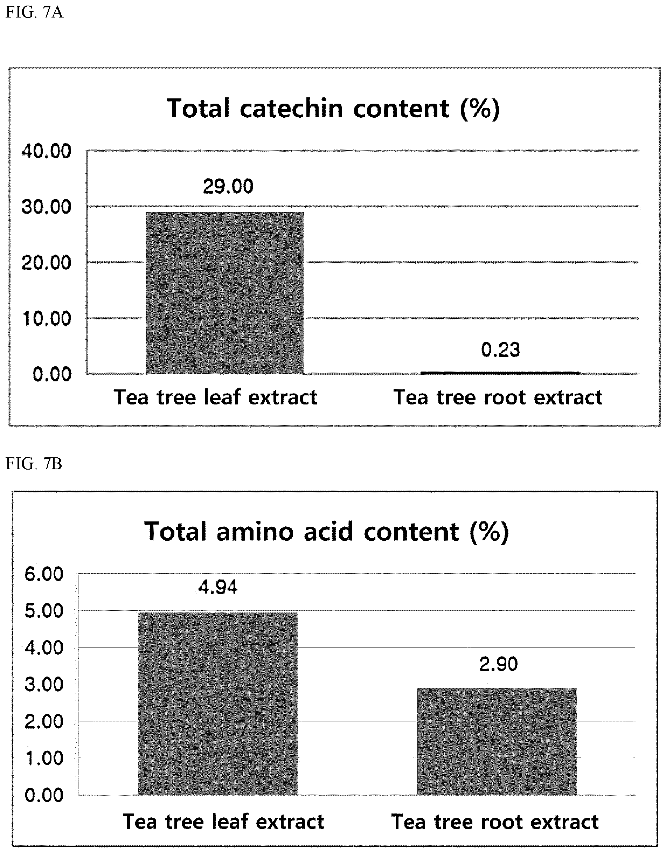

As a result, as shown in FIGs. 7A, 7B, Table 1 and Table 2, it was confirmed that the contents and components of catechin and amino acids contained in the tea tree leaf extract and the tea tree root extract were very different. Catechins, including epigallocatechin gallate (EGCG), were detected at a level of 0.2% in the tea tree root extract. In other words, since there is almost no EGCG in the tea tree root extract, it indicates that the senolytic effect does not result from EGCG.

**[Table 1]**

| **Component and content of catechin in tea tree leaf extract and tea tree root extract** | | |
|---|---|---|
| Item | Tea tree leaf (green tea) extract | Tea tree root extract |
| Gallocatechin | 0.3634 | ND |
| Epigallocatechin | 7.6432 | 0.0675 |
| Catechin | 0.2011 | 0.0306 |
| Epicatechin | 2.0375 | 0.0234 |
| Epigallocatechin gallate | 16.1052 | 0.1072 |
| Gallocatechin gallate | 0.0968 | 0.0018 |
| Epicatechin gallate | 2.5573 | ND |
| Catechin gallate | ND | ND |
| Total catechin content (%) | 29.0 | 0.23 |

**[Table 2]**

| **Component and content of amino acids in tea tree leaf extract and tea tree root extract** | | |
|---|---|---|
| Item | Tea tree leaf (green tea) extract | Tea tree root extract |
| Histidine | 110.732 | 3.174 |
| Asparagine | 387.195 | 131.104 |
| Serine | 1187.683 | 420.166 |
| Glutamine | 5035.244 | 1736.328 |
| Arginine | 1112.683 | 3802.368 |
| Glycine | 20.244 | 106.323 |
| Aspartic acid | 5123.537 | 733.765 |
| Glutamic acid | 6124.024 | 3523.438 |
| Threonine | 644.634 | 212.891 |
| Alanine | 795.244 | 3272.461 |
| γ-aminobutyric acid | ND | 2684.082 |
| Theanine | 26705.61 | 10269.287 |
| Proline | 252.927 | 1095.215 |
| Cystine | ND | ND |
| Lysine | ND | ND |
| Tyrosine | 369.024 | 146.973 |
| Methionine | ND | ND |
| Valine | ND | 294.067 |
| Leucine | 205.854 | 78.857 |
| Isoleucine | 234.39 | 199.707 |
| Phenylalanine | 418.78 | 106.567 |
| Tryptophan | 676.341 | 158.203 |
| Total amino acid content (µg/g) | 49404.146 | 28974.976 |
| Total amino acid content (%) | 4.94 | 2.90 |

### [Example 8]

### Measurement of cell toxicity by treatment with epigallocatechin gallate (EGCG)

Human fibroblasts were cultured in a 6-well plate culture medium. Senescent cells were prepared by inducing senescence by culturing the fibroblasts in DMEM (containing 10% FBS) medium containing doxorubicin (100 ng/ml) and insulin-like growth factor 1 (IGF-1) (100ng/ml) for 4 days. Then, the same medium was treated with a positive control material (ABT737, 1-10 uM) and the tea tree root extract (50-150ppm) and cultured for 72 hours. Young cells were treated with experimental materials in DMEM medium without inducing senescence and cultured for 72 hours.

In order to confirm the senolytic efficacy of the material treated with the tea tree root extract, the medium was replaced with a medium diluted 1/100 with Cell counting kit-8 (CCK-8, dojindo), cultured for 1 hour, and then the absorbance was measured at 540 nm.

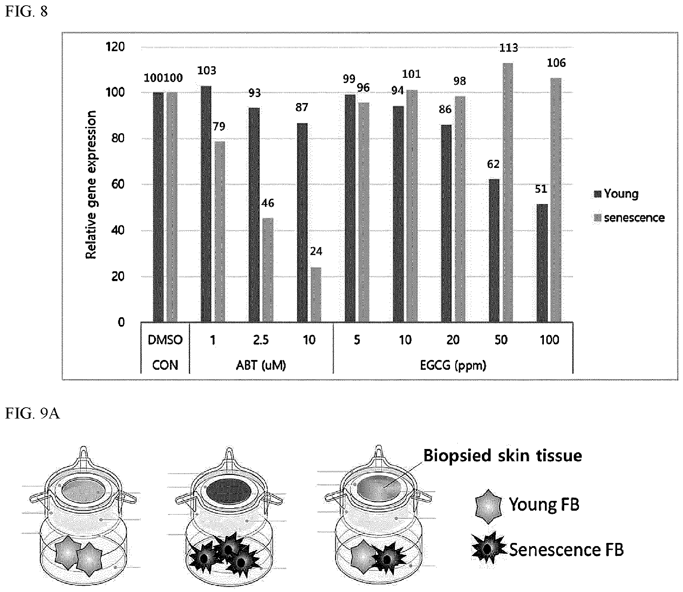

As shown in FIG. 8, it was confirmed that EGCG had no effect on killing senescent cells in fibroblasts and only showed cytotoxicity on young cells at a concentration of 50 ppm or higher.

### [Example 9]

### Confirmation of the efficacy of tea tree root extract in improving pigmentation in ex-vivo

Genoskin (GENOSKIN, Toulouse, France), made from human tissue derived from the abdomen of a 46-year-old woman, was purchased. Young or passage-aging human fibroblasts were treated with a positive control material (ABT737, 10 uM) and the tea tree root extract (100ppm) for 3 days. Then, these fibroblasts and Genoskin were cocultured for 7 days. Fontana-masson staining was performed to confirm melanin pigment, and changes in PEML, a melanocyte marker, and tyrosinase, a protein related to melanin synthesis, were confirmed.

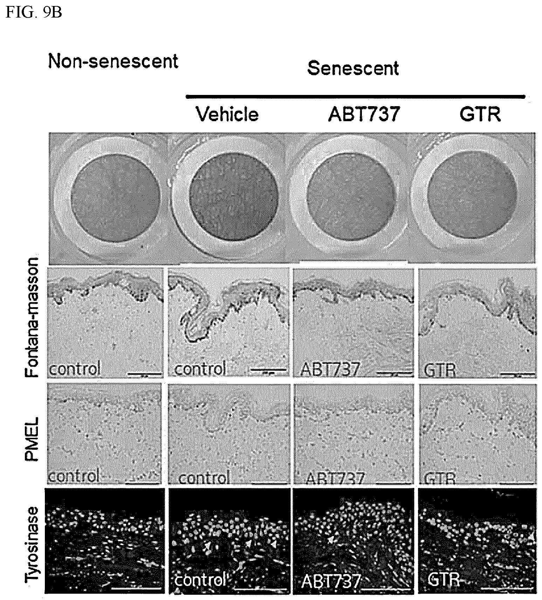

As shown in FIGs. 9A and 9B, it was confirmed that pigmentation of ex-vivo human skin increased due to aging fibroblasts, but this pigmentation was improved by treatment with the tea tree root extract. Through immunostaining, it was confirmed that melanin production was increased in surrounding melanocytes by aged fibroblasts, and that the melanin content that had been increased was decreased by the tea tree root extract. Additionally, there was no change in PEML, and the activity of tyrosinase was decreased by treatment with the tea tree root extract.

The disclosure is further illustrated by the following embodiments.

Embodiment 1. Use of a tea tree (*Camellia Sinensis*) root extract for a senotherapy; prevention or improvement of aging-related disease; improvement of skin elasticity or wrinkles; improvement of skin barrier; or improvement of skin pigmentation.

Embodiment 2. The use of Embodiment 1, wherein the senotherapy comprises a senolytic or senomorphic.

Embodiment 3. The use of Embodiment 1 or 2, wherein the senolytic or senomorphic is a skin cell senolytic or skin cell senomorphic.

Embodiment 4. The use of any one of Embodiments 1 to 3, wherein the skin cell is fibroblast.

Embodiment 5. The use of any one of Embodiments 1 to 4, wherein the senolytic is a senolytic based on apoptosis.

Embodiment 6. The use of any one of Embodiments 1 to 5, wherein the subject is characterized by a need for reduction of aging factor or Senescence Associated Secretory Phenotype (SASP) in skin cell.

Embodiment 7. The use of any one of Embodiment 1 to 6, wherein the aging factor is beta-galactosidase (β-Galactosidase).

Embodiment 8. The use of any one of Embodiments 1 to 7, wherein the SASP is one or more selected from the group consisting of IL-6, IL-8, MMP-1, and MCP-1.

Embodiment 9. The use of any one of Embodiments 1 to 8, wherein the tea tree root extract is an ethanol aqueous solution extract of the tea tree root.

Embodiment 10. The use of any one of Embodiments 1 to 9, wherein the use is non-pharmaceutical use.

Embodiment 11. A tea tree (*Camellia Sinensis*) root extract for use in prevention or treatment of aging-related disease.

Embodiment 12. The tea tree root extract of Embodiment 11, wherein the subject is characterized by a need for reduction of aging factor or Senescence Associated Secretory Phenotype (SASP) in skin cell.

Embodiment 13. The tea tree root extract of Embodiment 11 or 12, wherein the aging factor is beta-galactosidase (β-Galactosidase).

Embodiment 14. The tea tree root extract of any one of Embodiments 11 to 13, wherein the SASP is one or more selected from the group consisting of IL-6, IL-8, MMP-1, and MCP-1.

Embodiment 15. The tea tree root extract of any one of Embodiments 11 to 14, wherein the tea tree root extract is an ethanol aqueous solution extract of the tea tree root.

Embodiment 16. The tea tree root extract of any one of Embodiments 11 to 15, wherein the tea tree root extract is for pharmaceutical use.

## Claims

1. A non-therapeutic use of a tea tree (*Camellia Sinensis*) root extract for a senotherapy; prevention or improvement of aging-related disease; improvement of skin elasticity or wrinkles; improvement of skin barrier; or improvement of skin pigmentation.

2. The non-therapeutic use according to claim 1, wherein the senotherapy comprises a senolytic or senomorphic.

3. The non-therapeutic use according to claim 2, wherein the senolytic or senomorphic is a skin cell senolytic or skin cell senomorphic.

4. The non-therapeutic use according to claim 3, wherein the skin cell is fibroblast.

5. The non-therapeutic use according to claim 2 wherein the senolytic is a senolytic based on apoptosis.

6. The non-therapeutic use according to any one of the preceding claims, wherein the subject is **characterized by** a need for reduction of aging factor or Senescence Associated Secretory Phenotype (SASP) in skin cell.

7. The non-therapeutic use according to claim 6, wherein the aging factor is beta-galactosidase (β-Galactosidase).

8. The non-therapeutic use according to claim 6, wherein the SASP is one or more selected from the group consisting of IL-6, IL-8, MMP-1, and MCP-1.

9. The non-therapeutic use according to any one of the preceding claims, wherein the tea tree root extract is an ethanol aqueous solution extract of the tea tree root.

10. A tea tree (*Camellia Sinensis*) root extract for use in prevention or treatment of aging-related disease.

11. The tea tree root extract according to claim 10, wherein the subject is **characterized by** a need for reduction of aging factor or Senescence Associated Secretory Phenotype (SASP) in skin cell.

12. The tea tree root extract according to claim 11, wherein the aging factor is beta-galactosidase (β-Galactosidase).

13. The tea tree root extract according to claim 11, wherein the SASP is one or more selected from the group consisting of IL-6, IL-8, MMP-1, and MCP-1.

14. The tea tree root extract according to any one of claims 10 to 13, wherein the tea tree root extract is an ethanol aqueous solution extract of the tea tree root.
